# EUROPEAN PATENT APPLICATION

(11) **EP 4 542 214 A1**
(43) Date of publication of application: **23.04.2025**
(21) Application number: 24207636.2
(22) Date of filing: 18.10.2024
(51) Int. Cl.: G01N 27/327

(54) **BIOSENSOR**

(30) Priority: 19.10.2023 JP 2023180514
(71) Applicant: ARKRAY, Inc., Kyoto-shi, Kyoto 601-8045 (JP)
(72) Inventor: NAKATANI, Ayano, Kyoto, 602-0008 (JP)
(74) Representative: Dehns

(57) **Abstract**

According to an aspect, provided is a biosensor capable of highly accurate measurement. The present disclosure relates, in an aspect, to a biosensor comprising: a substrate with a conductive part provided on one main surface, the conductive part comprising an electrode pair having a working electrode and a counter electrode; a reagent layer provided on the working electrode and the counter electrode; and a flow channel for allowing a sample to flow from a sample supply port to the reagent layer; wherein the reagent layer has an average thickness of 4 µm to 12 µm; and wherein the reagent layer is provided to cover the entirety of the region of the working electrode within the flow channel and extend at least 50 µm from upstream and downstream edges of the working electrode, and to cover a region, of the region of the counter electrode within the flow channel, that corresponds to at least 70% of the region of the working electrode; and/or wherein the reagent layer is provided to cover the entirety of the surface of the working electrode within the flow channel and at least a portion of the surface of the counter electrode within the flow channel, and to extend upstream from an upstream edge of the working electrode and downstream from a downstream edge of the working electrode, with the extents of the upstream and downstream extensions each corresponding to at least 12.5% of the surface area of the working electrode within the flow channel, and a region of the counter electrode covered by the reagent layer corresponds to at least 70% of the surface area of the working electrode within the flow channel.

## Description

### BACKGROUND OF THE INVENTION

### 1. Field of the Invention

The present disclosure relates to a biosensor and a method for producing a biosensor.

### 2. Description of Related Art

Biosensors for measuring a measurement target in a sample, such as blood glucose in blood, typically have a pair of electrodes and a reagent layer formed thereon. When blood is supplied to the reagent layer of a biosensor, a reaction system is formed between the electrodes, thereby allowing measurement of the measurement target in the blood (WO 2003/025558).

The reagent layer is typically formed by applying a reagent solution containing oxidoreductase, an electron mediator, and the like onto the electrodes, and then allowing the reagent solution to dry. Examples of the method for applying the reagent solution include dipping, dispensing, screen printing, offset printing, inkjet printing, and the like (WO 2003/025558, JP 2011-99693A, JP H6-3317A, JP 2017-520773T).

### SUMMARY OF THE INVENTION

Biosensors typically have at least a substrate, a conductive part with a working electrode and a counter electrode formed on the substrate, and a reagent layer formed on the conductive part. The sizes of biosensors are typically very small, with lengths of several tens of millimeters in the longitudinal direction and lengths of a few millimeters in the lateral direction. Biosensors are used to measure a measurement target in a sample, such as blood glucose in blood, and are therefore required to ensure highly accurate measurement.

The measurement of a measurement target is performed as follows: in the reagent layer placed on the conductive part, the measurement target, such as blood glucose, contained in a sample, such as blood, reacts with a reagent in the reagent layer, and the reaction is detected. The present inventor found that the position of the reagent layer on the conductive part has a significant effect on the measurement accuracy, and that, in particular, the positional relationship between the reagent layer and the working electrode and the counter electrode, which constitute the conductive part, is extremely important.

The present disclosure provides a biosensor capable of highly accurate measurement.

In an aspect, the present disclosure relates to a biosensor comprising: a substrate with a conductive part provided on one main surface, the conductive part comprising an electrode pair having a working electrode and a counter electrode; a reagent layer provided on the working electrode and the counter electrode; and a flow channel for allowing a sample to flow from a sample supply port to the reagent layer, wherein the reagent layer is provided to cover the entirety of the region of the working electrode within the flow channel and extend at least 50 µm from upstream and downstream edges of the working electrode, and to cover a region, of the region of the counter electrode within the flow channel, that corresponds to at least 70% of the region of the working electrode within the flow channel, and the reagent layer has an average thickness of 4 µm to 12 µm.

In another aspect, the present disclosure relates to a biosensor comprising: a substrate with a conductive part provided on one main surface, the conductive part comprising an electrode pair having a working electrode and a counter electrode; a reagent layer provided on the working electrode and the counter electrode; and a flow channel for allowing a sample to flow from a sample supply port to the reagent layer, wherein the reagent layer is provided to cover the entirety of the surface of the working electrode within the flow channel and at least a portion of the surface of the counter electrode within the flow channel, and to extend upstream from an upstream edge of the working electrode and downstream from a downstream edge of the working electrode, with extents of the upstream and downstream extensions each corresponding to at least 12.5% of the surface area of the working electrode within the flow channel, and a region of the counter electrode covered by the reagent layer corresponds to at least 70% of the surface area of the working electrode within the flow channel, and the reagent layer has an average thickness of 4 µm to 12 µm.

In yet another aspect, the present disclosure relates to a method for producing the biosensor of the present disclosure, the method comprising preparing a substrate with a conductive part provided on one main surface, the conductive part comprising an electrode pair having a working electrode and a counter electrode, and forming a reagent layer on the working electrode and the counter electrode. In yet another aspect, the present disclosure relates to a method for producing a biosensor comprising a conductive part and a reagent layer within a flow channel, the method comprising: preparing a substrate with the conductive part formed on one main surface, the conductive part comprising an electrode pair having a working electrode and a counter electrode; and forming the reagent layer on the working electrode and the counter electrode, wherein the forming of the reagent layer includes dot printing a reagent solution such that the entirety of the region of the working electrode within the flow channel, regions extending at least 50 µm from upstream and downstream edges of the working electrode, and a region, of the region of the counter electrode within the flow channel, that corresponds to at least 70% of the region of the working electrode within the flow channel are covered by the reagent layer, and such that the reagent layer has an average thickness of 4 µm to 12 µm.

According to the present disclosure, it is possible to provide a biosensor capable of highly accurate measurement.

### BRIEF DESCRIPTION OF DRAWINGS

FIG. 1 is a schematic diagram showing a schematic configuration of a biosensor according to an embodiment of the present disclosure.
FIG. 2 is a schematic diagram showing the schematic configuration of the biosensor according to the embodiment of the present disclosure.
FIG. 3 is a graph showing an example of the results obtained in EXAMPLE (Glucose Current Value Measurement 1), illustrating the relationship between glucose concentration and reaction current values.
FIG. 4 is a graph showing an example of the results obtained in EXAMPLE (Glucose Current Value Measurement 1), illustrating the relationship between glucose concentration and reaction current values.
FIG. 5 is a graph showing an example of the results obtained in EXAMPLE (Glucose Current Value Measurement 1), illustrating reaction current values of various biosensors for a sample (glucose (GLU) concentration 3000 mg/dl × Hct 42%).
FIG. 6 is a graph showing an example of the results obtained in EXAMPLE (Glucose Current Value Measurement 2), illustrating reaction current values of various biosensors for a sample (glucose (GLU) concentration 1500 mg/dl × Hct 42%).

### DETAILED DESCRIPTION OF THE INVENTION

The biosensor of the present disclosure is used to measure a measurement target in a sample. In one or more embodiments, examples of the sample include a biological sample. In one or more embodiments, examples of the biological sample include: blood, urine, and samples derived therefrom; cell extract samples; and cell culture media. In one or more embodiments, examples of the measurement target include glucose, cholesterol, ethanol, sorbitol, fructose, cellobiose, lactic acid, and urea.

### (Biosensor)

An aspect of the present disclosure relates to a biosensor comprising: a substrate with a conductive part formed on one main surface, the conductive part comprising an electrode pair having a working electrode and a counter electrode; a reagent layer placed on the working electrode and the counter electrode; and a flow channel for allowing a sample to flow from a sample supply port to the reagent layer. In one aspect, the reagent layer of the biosensor of the present disclosure has an average thickness of 4 µm to 12 µm, and the reagent layer is formed to cover the entirety of the region of the working electrode within the flow channel and extend at least 50 µm from upstream and downstream edges of the working electrode, and to cover a region, of the region of the counter electrode within the flow channel, that corresponds to at least 70% of the region of the working electrode. In another aspect, the reagent layer of the biosensor of the present disclosure has an average thickness of 4 µm to 12 µm and is formed to cover the entirety of the surface of the working electrode within the flow channel and at least a portion of the surface of the counter electrode within the flow channel, and to extend upstream from an upstream edge of the working electrode and downstream from a downstream edge of the working electrode, with extents of the upstream and downstream extensions each corresponding to at least 12.5% of the surface area of the working electrode within the flow channel, and a region of the counter electrode covered by the reagent layer corresponds to at least 70% of the surface area of the working electrode within the flow channel.

In one or more embodiments, the biosensor of the present disclosure has a sample supply port and a flow channel that is in communication with the sample supply port, the sample supply port is provided for supplying a sample to the flow channel, and the flow channel is provided for allowing the sample supplied from the sample supply port to flow to the reagent layer within the flow channel. As used in the present disclosure, the term "upstream" refers to the upstream side in the direction of sample supply to the flow channel in the biosensor, that is, the sample supply port side, and the term "downstream" refers to the downstream side in the direction of sample supply, that is, the end side of the flow channel opposite to the sample supply port. As used in the present disclosure, the term "direction of sample supply" refers to the direction in which the sample supplied to the sample supply port of the biosensor flows in the flow channel.

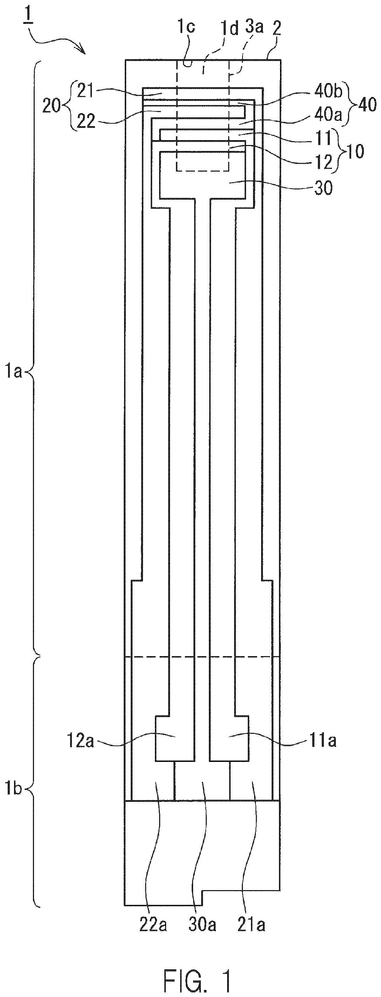

The reagent layer of a biosensor is typically formed by applying a reagent solution onto the conductive part through screen printing or the like, considering convenience and efficiency. On the other hand, with this method, to produce a biosensor that provides sufficient measurement accuracy, it was considered necessary to form the reagent layer such that the surfaces of the electrodes (working electrode and counter electrode) within the flow channel are entirely covered by the reagent (see, for example, WO 2003/025558 (FIG. 1), JP 2017-520773T (FIG. 1), etc.).

However, the present inventor found that by forming a reagent layer with an average thickness of 4 µm to 12 µm through dot printing or the like, for example, it is possible to achieve highly accurate measurement even when the surfaces of the electrodes within the flow channel are not entirely covered by the reagent. Furthermore, the present inventor also found that to achieve highly accurate measurement, it is important to cover the edges of the working electrode and also certain areas extending from the edges to predetermined regions with the reagent layer.

According to the biosensor of the present disclosure, the reagent layer has an average thickness of 4 µm to 12 µm, and the reagent layer is formed to extend not only over the entire surface of the working electrode within the flow channel but also over predetermined areas from the edges of the working electrode, and furthermore, to cover a region of the surface of the counter electrode within the flow channel that corresponds to at least 70% of the region of the working electrode within the flow channel. Thus, highly accurate measurement of an analyte can be achieved.

As used in the present disclosure, the phrase "the reagent layer covers the entirety of the region of the working electrode within the flow channel" means that substantially the entire surface of the working electrode exposed within the flow channel is required to be covered by the reagent. The condition in which substantially the entire surface of the working electrode within the flow channel is covered by the reagent, according to the present disclosure, is satisfied in one or more embodiments when 96% or more, 97% or more, 98% or more, 99% or more, 99.9% or more, 99.99% or more, or 100% of the surface of the working electrode within the flow channel is covered by the reagent.

The reagent layer is formed to cover the entire region (surface) of the working electrode within the flow channel and also extend from the upstream edge and the downstream edge of the working electrode. As used in the present disclosure, the phrase "the reagent layer extends from the edges of the working electrode" means that the reagent layer is formed on a surface of the substrate (or on a surface of a component part formed on the substrate) so as to extend past (spread from) the edges of the working electrode. Therefore, according to the present disclosure, the edges of the working electrode within the flow channel are covered by the reagent layer.

In one or more embodiments, the reagent layer extends at least 50 µm from the upstream and downstream edges of the working electrode. From the viewpoint of improving measurement accuracy even further, in one or more embodiments, it is preferable that the reagent layer extends 60 µm or more, 70 µm or more, 80 µm or more, 90 µm or more, 100 µm or more, 125 µm or more, 150 µm or more, 175 µm or more, 200 µm or more, 225 µm or more, 250 µm or more, 275 µm or more, or 300 µm or more. The extent to which the reagent layer extends from each of the upstream and downstream edges of the working electrode is, in one or more embodiments, 500 µm or less, 450 µm or less, or 400 µm or less.

The extent to which the reagent layer extends from the upstream edge of the working electrode is, in one or more embodiments, 50 µm or more and 500 µm or less, 50 µm or more and 450 µm or less, 50 µm or more and 400 µm or less, 50 µm or more and 350 µm or less, 50 µm or more and 340 µm or less, 50 µm or more and 330 µm or less, 50 µm or more and 320 µm or less, or 50 µm or more and 310 µm or less. The extent to which the reagent layer extends from the downstream edge of the working electrode is, in one or more embodiments, 175 µm or more and 500 µm or less, 200 µm or more and 500 µm or less, 225 µm or more and 500 µm or less, 250 µm or more and 500 µm or less, 275 µm or more and 500 µm or less, 275 µm or more and 450 µm or less, 275 µm or more and 440 µm or less, 275 µm or more and 430 µm or less, or 280 µm or more and 420 µm or less. From the same viewpoint, the extent to which the reagent layer extends from each of the upstream and downstream edges of the working electrode is preferably 100 µm or more and 450 µm or less, or 100 µm or more and 150 µm or less.

In one or more embodiments, the extent to which the reagent layer extends from the upstream edge of the working electrode is 50 µm or more and 500 µm or less, and the extent to which the reagent layer extends from the downstream edge of the working electrode is 175 µm or more and 500 µm or less. In one or more embodiments, the extent to which the reagent layer extends from the upstream edge of the working electrode is 50 µm or more and 450 µm or less, and the extent to which the reagent layer extends from the downstream edge of the working electrode is 200 µm or more and 500 µm or less. In one or more embodiments, the extent to which the reagent layer extends from the upstream edge of the working electrode is 50 µm or more and 400 µm or less, and the extent to which the reagent layer extends from the downstream edge of the working electrode is 225 µm or more and 500 µm or less. In one or more embodiments, the extent to which the reagent layer extends from the upstream edge of the working electrode is 50 µm or more and 350 µm or less, and the extent to which the reagent layer extends from the downstream edge of the working electrode is 250 µm or more and 500 µm or less. In one or more embodiments, the extent to which the reagent layer extends from the upstream edge of the working electrode is 50 µm or more and 340 µm or less, and the extent to which the reagent layer extends from the downstream edge of the working electrode is 275 µm or more and 500 µm or less. In one or more embodiments, the extent to which the reagent layer extends from the upstream edge of the working electrode is 50 µm or more and 330 µm or less, and the extent to which the reagent layer extends from the downstream edge of the working electrode is 275 µm or more and 450 µm or less. In one or more embodiments, the extent to which the reagent layer extends from the upstream edge of the working electrode is 50 µm or more and 320 µm or less, and the extent to which the reagent layer extends from the downstream edge of the working electrode is 275 µm or more and 440 µm or less. In one or more embodiments, the extent to which the reagent layer extends from the upstream edge of the working electrode is 50 µm or more and 310 µm or less, and the extent to which the reagent layer extends from the downstream edge of the working electrode is 275 µm or more and 430 µm or less, or 280 µm or more and 420 µm or less.

The distance from the upstream edge of the working electrode to the upstream edge of the reagent layer is, in one or more embodiments, 50 µm or more and 500 µm or less, 50 µm or more and 450 µm or less, 50 µm or more and 400 µm or less, 50 µm or more and 350 µm or less, 50 µm or more and 340 µm or less, 50 µm or more and 330 µm or less, 50 µm or more and 320 µm or less, or 50 µm or more and 310 µm or less. The distance from the downstream edge of the working electrode to the downstream edge of the reagent layer is, in one or more embodiments, 175 µm or more and 500 µm or less, 200 µm or more and 500 µm or less, 225 µm or more and 500 µm or less, 250 µm or more and 500 µm or less, 275 µm or more and 500 µm or less, 275 µm or more and 450 µm or less, 275 µm or more and 440 µm or less, 275 µm or more and 430 µm or less, or 280 µm or more and 420 µm or less.

In one or more embodiments, the extent to which the reagent layer extends from each of the upstream and downstream edges of the working electrode is within a range of at least 12.5% of the surface area of the working electrode within the flow channel. From the viewpoint of improving measurement accuracy even further, in one or more embodiments, it is within a range of 13% or more, 14% or more, 15% or more, 20% or more, 25% or more, 30% or more, 35% or more, 40% or more, 45% or more, 50% or more, 55% or more, 60% or more, 70% or more, 80% or more, 90% or more, or 100% of the surface area of the working electrode within the flow channel.

The extent to which the reagent layer extends from the upstream edge of the working electrode is, in one or more embodiments, 12.5% or more and 100% or less, 12.5% or more and 90% or less, 12.5% or more and 85 % or less, 12.5% or more and 80% or less, or 13% or more and 75% or less of the surface area of the working electrode within the flow channel.

The extent to which the reagent layer extends from the downstream edge of the working electrode is, in one or more embodiments, 45% or more and 100% or less, 50% or more and 100% or less, 55% or more and 100% or less, 60% or more and 100% or less, 65% or more and 100% or less, 70% or more and 100% or less, 75% or more and 100% or less of the surface area of the working electrode within the flow channel.

In one or more embodiments, the extent to which the reagent layer extends from the upstream edge of the working electrode is 12.5 % or more and 100% or less of the surface area of the working electrode within the flow channel, and the extent to which the reagent layer extends from the downstream edge of the working electrode is 45% or more and 100% or less of the surface area of the working electrode within the flow channel. In one or more embodiments, the extent to which the reagent layer extends from the upstream edge of the working electrode is 12.5% or more and 90% or less of the surface area of the working electrode within the flow channel, and the extent to which the reagent layer extends from the downstream edge of the working electrode is 50% or more and 100% or less of the surface area of the working electrode within the flow channel. In one or more embodiments, the extent to which the reagent layer extends from the upstream edge of the working electrode is 12.5% or more and 85% or less of the surface area of the working electrode within the flow channel, and the extent to which the reagent layer extends from the downstream edge of the working electrode is 55% or more and 100% or less of the surface area of the working electrode within the flow channel. In one or more embodiments, the extent to which the reagent layer extends from the upstream edge of the working electrode is 12.5% or more and 80% or less of the surface area of the working electrode within the flow channel, and the extent to which the reagent layer extends from the downstream edge of the working electrode is 60% or more and 100% or less of the surface area of the working electrode within the flow channel. In one or more embodiments, the extent to which the reagent layer extends from the upstream edge of the working electrode is 13% or more and 75% or less of the surface area of the working electrode within the flow channel, and the extent to which the reagent layer extends from the downstream edge of the working electrode is 65% or more and 100% or less of the surface area of the working electrode within the flow channel. In one or more embodiments, the extent to which the reagent layer extends from the upstream edge of the working electrode is 13% or more and 75% or less of the surface area of the working electrode within the flow channel, and the extent to which the reagent layer extends from the downstream edge of the working electrode is 70% or more and 100% or less of the surface area of the working electrode within the flow channel. In one or more embodiments, the extent to which the reagent layer extends from the upstream edge of the working electrode is 13% or more and 75% or less of the surface area of the working electrode within the flow channel, and the extent to which the reagent layer extends from the downstream edge of the working electrode is 75% or more and 100% or less of the surface area of the working electrode within the flow channel.

From the viewpoint of improving measurement accuracy even further, it is preferable in one or more embodiments that the reagent layer extending from the downstream edge of the working electrode has a larger surface area than the reagent layer extending from the upstream edge of the working electrode. In one or more embodiments, the surface area of the reagent layer extending from the downstream edge of the working electrode is at least 1.3 times, at least 1.4 times, at least 1.5 times, at least 2 times, at least 2.5 times, at least 3 times, or at least 4 times larger than the surface area of the reagent layer extending from the upstream edge of the working electrode.

The reagent layer is formed to cover at least a portion of the region (surface) of the counter electrode within the flow channel. The reagent layer is formed to cover a portion of the region (surface) of the counter electrode within the flow channel, that corresponds to at least 70% of the region (area) of the working electrode within the flow channel. From the viewpoint of improving measurement accuracy even further, in one or more embodiments, this region may correspond to 75% or more, 80% or more, 85% or more, 90% or more, 95% or more, 96% or more, 97% or more, 98% or more, 99% or more, or 100% of the region (area) of the working electrode within the flow channel. In one or more embodiments, the region (area) of the reagent layer that covers the counter electrode may correspond to 100%, 99% or less, 98% or less, 97% or less, 96% or less, 95% or less, 94% or less, 93% or less, 92% or less, 91% or less, 90% or less, 89% or less, 88% or less, 87% or less, 86% or less, or 85% or less of the region (area) of the working electrode within the flow channel. In one or more embodiments, the region (area) of the reagent layer that covers the counter electrode may correspond to 70% or more and 100% or less, 75% or more and 100% or less, 80% or more and 100% or less, 85% or more and 100% or less, 90% or more and 100% or less, or 95% or more and 100% or less of the region (area) of the working electrode within the flow channel.

In addition, the region of the reagent layer that covers the counter electrode can be, in one or more embodiments, at least 70% of the region (area) of the counter electrode within the flow channel. From the viewpoint of improving measurement accuracy even further, in one or more embodiments, the region of the reagent layer that covers the counter electrode may be 75% or more, 80% or more, 85% or more, 90% or more, 95% or more, 96% or more, 97% or more, 98% or more, 99% or more, or 100% of the region (area) of the counter electrode within the flow channel. In one or more embodiments, the region of the reagent layer that covers the counter electrode may be 100%, 99% or less, 98% or less, 97% or less, 96% or less, 95% or less, 94% or less, 93% or less, 92% or less, 91 % or less, 90% or less, 89% or less, 88% or less, 87% or less, 86% or less, or 85% or less of the region (area) of the counter electrode within the flow channel.

In one or more embodiments, the region of the reagent layer that covers the counter electrode may be 70% or more and 100% or less, 75% or more and 100% or less, 80% or more and 100% or less, 85% or more and 100% or less, 90% or more and 100% or less, or 95% or more and 100% or less of the region (area) of the counter electrode within the flow channel.

The reagent layer has an average thickness of 4 µm to 12 µm. From the viewpoint of improving measurement accuracy even further, the average thickness of the reagent layer is preferably 5 µm or more, 6 µm or more, 7 µm or more, 8 µm or more, 9 µm or more, or 9.5 µm or more. In one or more embodiments, the average thickness of the reagent layer is 5 µm or more and 12 µm or less, 6 µm or more and 12 µm or less, 7 µm or more and 12 µm or less, 8 µm or more and 12 µm or less, 9 µm or more and 12 µm or less, or 9.5 µm or more and 12 µm or less. In one or more embodiments, the average thickness of the reagent layer is 4 µm or more and 11 µm or less, 4 µm or more and 10 µm or less, 4 µm or more and 9 µm or less, 4 µm or more and 8 µm or less, or 5 µm or more and 8 µm or less. The average thickness of the reagent layer can be measured and calculated using a method described in EXAMPLE.

In one or more embodiments, the reagent layer is preferably formed through dot printing. With dot printing, a reagent layer of the above-described average thickness can be easily formed by repeatedly performing the operation of allowing ejected droplets of the reagent solution to dry, ejecting additional droplets of the reagent solution onto the dried droplets, and then allowing the newly ejected droplets to dry, as will be described later. The formation of the reagent layer by dot printing can be performed by referring to a method described in EXAMPLE, the method disclosed in EP 4266037A or the method disclosed in US 2023/338945A. The content of these documents is hereby incorporated by reference as part of the present disclosure.

The size (area) of the reagent layer within the flow channel can be appropriately determined based on the shapes of the substrate, flow channel, and conductive part, for example. When the substrate has a rectangular shape with a length of 7 mm in the lateral direction and a length of 30 mm in the longitudinal direction, the flow channel has a rectangular shape with a width (length in the lateral direction of the substrate) of 1.8 mm and a length (length in the longitudinal direction of the substrate) of 4 mm, and the working electrode within the flow channel has a rectangular shape with a width (length in the lateral direction of the substrate) of 1.8 mm and a length (length within the longitudinal direction of the substrate) of 0.8 mm, the size of the reagent layer, in one or more embodiments, includes a width (length in the lateral direction of the substrate) of 1.5 mm or more, 2 mm or more, 2.5 mm or more, or 3 mm or more, and/or 5 mm or less, 4.5 mm or less, or 4 mm or less, and/or a length (length in the longitudinal direction of the substrate) of 0.5 mm or more, 0.6 mm or more, 0.8 mm or more, or 0.9 mm or more, and/or 2 mm or less, 1.6 mm or less, or 1.4 mm or less. When the substrate has a rectangular shape with a length of 7 mm in the lateral direction and a length of 30 mm in the longitudinal direction, the flow channel has a rectangular shape with a width (length in the lateral direction of the substrate) of 1.8 mm and a length (length in the longitudinal direction of the substrate) of 4 mm, and the working electrode within the flow channel has a rectangular shape with a width (length in the lateral direction of the substrate) of 1.8 mm and a length (length within the longitudinal direction of the substrate) of 0.8 mm, in one or more embodiments, the size of the reagent layer includes, a width (length in the lateral direction of the substrate) of 1.5 mm or more and 5 mm or less, 2 mm or more and 5 mm or less, 2.5 mm or more and 4.5 mm or less, or 3 mm or more and 4 mm or less, and/or a length (length in the longitudinal direction of the substrate) of 0.5 mm or more and 2 mm or less, 0.6 mm or more and 2 mm or less, 0.8 mm or more and 1.6 mm or less, or 0.9 mm or more and 1.4 mm or less.

In one or more embodiments, an example of the substrate is a sheet-like insulating substrate. In one or more embodiments, examples of the material of the substrate include thermoplastic resins, thermosetting resins, glass, ceramic, and paper. In one or more embodiments, polyetherimide (PEI), polyethylene terephthalate (PET), polyethylene (PE), polybutylene terephthalate (PBT), polystyrene (PS), polymethacrylate (PMMA), polypropylene (PP), polyimide resins, acrylic resins, epoxy resins, glass epoxy, and the like can be used as the material.

The conductive part includes at least one electrode pair having a working electrode and a counter electrode and is provided on one main surface of the substrate. When the conductive part includes a plurality of electrode pairs, the conductive part may have, for example, a first electrode pair for measuring a first measurement target (e.g., blood glucose) and a second electrode pair for measuring a second measurement target (e.g., hematocrit), with both measurement targets being contained in the same sample, such as blood. Furthermore, the first electrode pair and the second electrode pair may share a common counter electrode. Here, when the conductive part has a plurality of electrode pairs, the reagent layer included in the biosensor of the present disclosure only needs to be provided on at least one electrode pair, or it may be provided on multiple electrode pairs. Also, in one or more embodiments, the conductive part may further have a sensing electrode.

In one or more embodiments, the working electrode and the counter electrode extend parallel to a direction perpendicular to the direction of sample supply in the flow channel. In one or more embodiments, the working electrode is provided upstream of the counter electrode and is preferably provided upstream of and adjacent to the counter electrode.

In one or more embodiments, a conductive material can be used as the material of the electrodes. In one or more embodiments, examples of the conductive material include a metal material and a carbon material. In one or more embodiments, examples of the metal material include gold (Au), platinum (Pt), silver (Ag), ruthenium (Ru), palladium (Pd), and nickel (Ni) alloys. In one or more embodiments, examples of the nickel alloys include nickel-vanadium alloys, nickel-tungsten alloys, and nickel-ruthenium alloys. In one or more embodiments, examples of the carbon material include graphite, carbon nanotubes, graphene, and mesoporous carbon.

In one or more embodiments, the electrodes may be thin-film electrodes or the like formed by depositing a film of the above-described material, such as a metal material, on the substrate. In one or more embodiments, the film may be formed using a method such as screen printing, physical vapor deposition (PVD, e.g., sputtering), or chemical vapor deposition (CVD).

In one or more embodiments, the working electrode and the counter electrode may be insulated from each other. When the electrodes constituting the conductive part are formed through physical vapor deposition using a metal material, in one or more embodiments, the electrodes may be insulated from each other by drawing a predetermined electrode pattern with a laser beam (trimming). When the conductive part is formed using a carbon material, in one or more embodiments, an insulating portion may be formed by spacing the electrodes apart by a predetermined distance.

In one or more embodiments, the biosensor of the present disclosure may also include a spacer placed on the substrate and a cover placed on the spacer. In one or more embodiments, the spacer may have a notch. Placing the spacer on the substrate such that the conductive part and the reagent layer are positioned inside the notch of the spacer allows a space formed by the substrate, the notch, and the cover to serve as the sample supply port and flow channel.

Examples of the shape of the notch of the spacer include U-shapes with angular corners or with curved corners. There is no particular limitation on the material of the spacer, and materials similar to those of the substrate can be used.

In one or more embodiments, the cover may have an air hole. Preferably, the cover is placed on the spacer such that the air hole is positioned directly above the notch of the spacer. There is no particular limitation on the material of the cover, and materials similar to those of the substrate may be used. A hydrophilic film with a contact angle (hydrophobicity) higher than that of the spacer is preferable.

In the biosensor of the present disclosure, in one or more embodiments, the reagent layer is required to be formed on the conductive part (in particular, one electrode pair having the working electrode and the counter electrode) located inside the flow channel formed by the above-described notch of the spacer (i.e., located within the notch). The reagent layer may also be formed on the conductive part that is located outside the flow channel. In other words, the reagent layer may be formed to extend outside the notch (flow channel) of the spacer, that is, past an end (boundary) of the flow channel. Note that, since the reagent layer formed outside the notch (flow channel) of the spacer does not substantially contact the sample, the reagent in that portion of the reagent layer does not contribute to (influence) the measurement, and therefore, the shape, size, thickness, and the like of the reagent layer formed outside the notch (flow channel) of the spacer are not particularly limited.

In one or more embodiments, examples of the reagent contained in the reagent layer include oxidoreductase and an electron mediator.

### (Oxidoreductase)

In one or more embodiments, examples of the oxidoreductase include glucose dehydrogenase (GDH), glucose oxidase (GOD), cholesterol oxidase, quinoheme ethanol dehydrogenase (QHEDH (PQQ Ethanol dh)), sorbitol dehydrogenase, D-fructose dehydrogenase, D-glucoside-3-dehydrogenase, cellobiose dehydrogenase, lactate oxidase (LOD), lactate dehydrogenase (LDH), and urate dehydrogenase.

In one or more embodiments, the oxidoreductase may contain, as a coenzyme (also referred to as a catalytic subunit or catalytic domain), flavin adenine dinucleotide (FAD), pyrroloquinoline quinone (PQQ), nicotinamide adenine dinucleotide (NAD), or nicotinamide adenine dinucleotide phosphate (NADP). In one or more embodiments, examples of the oxidoreductase containing a coenzyme include FAD-GDH, PQQ-GDH, NAD-GDH, and NADP-GDH.

In one or more embodiments, examples of the oxidoreductase include Aspergillus oryzae-type FAD-GDH (flavin adenine dinucleotide-dependent glucose dehydrogenase or flavin adenine dinucleotide-binding glucose dehydrogenase). In one or more embodiments, an Aspergillus oryzae-type FAD-GDH disclosed in JP 2013-083634A can be used as the Aspergillus oryzae-type FAD-GDH. The content of this document is hereby incorporated by reference as part of the present disclosure.

In one or more embodiments, the amount of oxidoreductase added in the reagent layer is 1000 KU/cm³ or less, preferably 500 KU/cm³ or less, and more preferably 300 KU/cm³ or less. The lower limit of the amount of oxidoreductase added in the reagent layer is not particularly limited, and is 1 KU/cm³ or more in one or more embodiments. In one or more embodiments, the amount of oxidoreductase added in the reagent layer is 200 KU/cm³ to 300 KU/cm³, preferably 200 KU/cm³ to 280 KU/cm³, and more preferably 210 KU/cm³ to 260 KU/cm³. As used in the present disclosure, "U" represents a unit of enzyme activity and refers to the amount of enzyme that acts on 1 µmol of substrate per minute under optimal conditions.

### (Electron Mediator)

In one or more embodiments, examples of the electron mediator include a ruthenium compound, 1-Methoxy-PES (1-Methoxy-5-ethylphenazinium ethylsulfate, 1-mPES), 1-Methoxy-PMS (1-Methoxy-5-methylphenazinium methylsulfate, 1-mPMS), a phenylenediamine compound, a quinone compound, a ferricyanide compound, Coenzyme Q0 (2,3-Dimethoxy-5-methyl-p-benzoquinone), AZURE A Chloride (3-amino-7-(dimethylamino)phenothiazin-5-ium chloride), Phenosafranin (3,7-Diamino-5-phenylphenanzinium chloride), 6-Aminoquinoxaline, and Tetrathiafulvalene.

In one or more embodiments, a ruthenium compound that is present in a reaction system as an oxidized ruthenium complex and functions as an electron mediator can be used as the ruthenium compound. There is no particular limitation on the type of ligand of the ruthenium complex. In one or more embodiments, examples of the ruthenium compound include oxidized ruthenium complexes represented by the chemical formula below:

[Ru(NH₃)₅X]ⁿ⁺

where X represents NH₃, a halogen ion, CN, pyridine, nicotinamide, or H₂O, with NH₃ or a halogen ion being preferable among these. In one or more embodiments, examples of the halogen ion include Cl⁻, F⁻, Br⁻, and I⁻. In the above formula, ⁿ⁺ represents the valence of the oxidized ruthenium (III) complex that is determined by the type of X. In one or more embodiments, a ruthenium complex disclosed in JP 2018-013400A can be used as the ruthenium complex. The content of this document is hereby incorporated by reference as part of the present disclosure. In one or more embodiments, examples of the ruthenium compound include ruthenium hexamine such as [Ru(NH₃)₆]²⁺ or [Ru(NH₃)₆]³⁺. In one or more embodiments, examples of the ruthenium compound include [Ru(NH₃)₆]Cl₃.

In one or more embodiments, examples of the phenylenediamine compound include N,N-Dimethyl-1,4-phenylenediamine and N,N,N',N'-tetramethyl-1,4-phenylenediaminedihydrochloride.

In one or more embodiments, examples of the quinone compound include 1,4-Naphthoquinone, 2-Methyl-1,4-Naphthoquinone (VK3), 9,10-Phenanthrenequinone, 1,2-Naphthoquinone, p-Xyloquinone, Methylbenzoquinone, 2,6-Dimethylbenzoquinone, Sodium1,2-Naphthoquinone-4-sulfonate, 1,4-Anthraquinone, Tetramethylbenzoquinone, and Thymoquinone.

In one or more embodiments, examples of the ferricyanide compound include calcium ferricyanide.

In one or more embodiments, the amount of electron mediator added in the reagent layer is 0.1 mmol/cm³ or more, preferably 0.5 mmol/cm³ or more, and more preferably 1 mmol/cm³ or more. The upper limit of the amount of electron mediator added in the reagent layer is not particularly limited, and is 50 mmol/cm³ or less or 10 mmol/cm³ or less in one or more embodiments. In one or more embodiments, the amount of electron mediator added in the reagent layer is 0.1 mmol/cm³ or more and 50 mmol/cm³ or less, preferably 0.5 mmol/cm³ or more and 50 mmol/cm³ or less, and more preferably 1 mmol/cm³ or more and 10 mmol/cm³ or less.

In one or more embodiments, the reagent layer may also contain an additional component in addition to the oxidoreductase and the electron mediator. In one or more embodiments, examples of the additional component include a buffer, an amino acid, a surfactant, an antifoaming agent, and a binder.

In one or more embodiments, examples of the buffer include a phosphate buffer, an amine-based buffer, and a buffer having a carboxyl group. In one or more embodiments, examples of the amine-based buffer include Tris (tris(hydroxymethyl)aminomethane), ACES (N-(2-Acetamido)-2-aminoethanesulfonic acid), CHES (N-Cyclohexyl-2-aminoethanesulfonic acid), CAPSO (3-(Cyclohexylamino)-2-hydroxy-1-propanesulfonic acid), TAPS (N-Tris(hydroxymethyl)methyl-3-aminopropanesulfonic acid), CAPS (N-Cyclohexyl-3-aminopropanesulfonic acid), Bis-Tris (Bis(2-hydroxyethyl)iminotris(hydroxymethyl)methane), TAPSO (2-Hydroxy-N-tris(hydroxymethyl)methyl-3-aminopropanesulfonic acid), TES (N-Tris(hydroxymethyl)methyl-2-aminoethanesulfonic acid), Tricine (N-[Tris(hydroxymethyl)methyl]glycine), and ADA (N-(2-Acetamido)iminodiacetic acid). In one or more embodiments, examples of the buffer having a carboxyl group include a citrate buffer, a phosphate-citrate buffer, an acetic acid-sodium acetate buffer, a malic acid-sodium acetate buffer, a malonic acid-sodium acetate buffer, and a succinic acid-sodium acetate buffer. One type of buffer may be used alone, or two or more types of buffers may be used in combination. In one or more embodiments, the buffer has a pH of 6.8 to 7.5, and preferably 6.9 to 7.0.

In one or more embodiments, examples of the amino acid include glycine, serine, lysine, and histidine.

In one or more embodiments, examples of the surfactant include TritonX-100 ((p-tert-Octylphenoxy)polyethoxyethanol), Tween20 (Polyoxyethylene Sorbitan Monolaurate), sodium dodecyl sulfate, perfluorooctanesulfonic acid, sodium stearate, alkylaminocarboxylic acid (or salts thereof), carboxybetaine, sulfobetaine, and phosphobetaine. One type of surfactant may be used alone, or two or more types of surfactants may be used in combination.

In one or more embodiments, examples of the binder include a resin binder and a layered inorganic compound. In one or more embodiments, examples of the resin binder include a butyral resin binder and a polyester resin binder. A layered inorganic compound disclosed in WO 2005/043146 can be used as the layered inorganic compound. In one or more embodiments, examples of the layered inorganic compound include swellable clay minerals with ion exchange capacity. In one or more embodiments, examples of the layered inorganic compound include bentonite, smectite, vermiculite, and synthetic fluorine mica. One type of binder may be used alone, or two or more types of binders may be used in combination.

In one or more embodiments, the biosensor of the present disclosure can be produced by preparing a substrate with a conductive part provided on one main surface, the conductive part including at least one electrode pair having a working electrode and a counter electrode, and then dot printing a reagent solution onto the conductive part so as to form a reagent layer at the position specified above.

### (Method for Producing Biosensor)

In another aspect, the present disclosure relates to a method for producing a biosensor. In another aspect of the method for producing a biosensor is a method for producing a biosensor of the present disclosure, the method comprising preparing a substrate with a conductive part provided on one main surface, the conductive part comprising an electrode pair having a working electrode and a counter electrode, and forming a reagent layer on the working electrode and the counter electrode.

In another aspect of the method for producing a biosensor is a method for producing a biosensor comprising a conductive part and a reagent layer within a flow channel, the method comprising: preparing a substrate with the conductive part provided on one main surface, the conductive part comprising an electrode pair having a working electrode and a counter electrode; and forming the reagent layer on the working electrode and the counter electrode, wherein the forming of the reagent layer includes dot printing a reagent solution such that the entirety of the region of the working electrode within the flow channel, regions extending at least 50 µm from upstream and downstream edges of the working electrode, and a region, of the region of the counter electrode within the flow channel, that corresponds to at least 70% of the region of the working electrode within the flow channel are covered by a reagent, and such that the reagent layer has an average thickness of 4 µm to 12 µm.

In another aspect of the method for producing a biosensor of the present disclosure, the forming of the reagent layer includes dot printing the reagent solution to cover the entirety of the surface of the working electrode within the flow channel and at least a portion of the surface of the counter electrode within the flow channel, the dot printing including dot printing a reagent solution onto areas extending upstream from the upstream edge of the working electrode and downstream from the downstream edge of the working electrode, respectively, with these areas each corresponding to at least 12.5% of the surface area of the working electrode within the flow channel, and dot printing onto an area, of the surface of the counter electrode within the flow channel, that corresponds to at least 70% of the surface area of the working electrode within the flow channel.

According to the production method of the present disclosure, the reagent layer is formed by dot printing the reagent solution onto the above-described predetermined areas on the conductive part. This enables the production of the biosensor of the present disclosure, which is capable of highly accurate measurement. For this reason, in one or more embodiments, the production method of the present disclosure can also be said to be a method for producing the biosensor of the present disclosure.

In one or more embodiments, the dot printing includes repeating the operation of ejecting droplets of the reagent solution onto the conductive part, allowing the ejected droplets to dry, and then ejecting additional droplets of the reagent solution onto the dried droplets. This allows the reagent to be stacked three-dimensionally to form the reagent layer with an average thickness of 4 µm to 12 µm. In one or more embodiments, the dot printing can be performed using an inkjet printer or the like. The composition of the reagent solution is as described above.

In one or more embodiments, the ejected amount of the reagent solution can be appropriately determined depending on the pitch of the positions where droplets are ejected, the size of the dried droplet to be formed, and the like. In one or more embodiments, the ejected amount of the reagent solution is 10 ng to 30 ng per one droplet, and from the viewpoint of further reducing irregularities in the concentrations and amounts of the components in the reagent layer and further enhancing the reproducibility of the measurement accuracy of a biosensor to be obtained, the foregoing amount is 12 ng to 25 ng, or 12 ng to 15 ng.

In one or more embodiments, the ejected amount of the reagent solution per one droplet may be different between the first layer and the subsequent layers. From the viewpoint of further reducing irregularities in the concentrations and amounts of reagent components in the reagent layer, and further enhancing the reproducibility of the measurement accuracy of a biosensor to be obtained, the ejected amount of the reagent solution for the second or subsequent layer is preferably greater than the ejected amount of the reagent solution for the first layer. In one or more non-limiting embodiments, for example, the ejected amount of the reagent solution for the first layer is set to less than 20 ng per one droplet, and the ejected amount of the reagent solution for the second or subsequent layer is set to 20 ng or more per one droplet. In one or more embodiments, the ejected amount for the reagent solution for the first layer is 10 ng to 18 ng per one droplet, and from the viewpoint of further reducing irregularities in the concentrations and amounts of reagent components in the reagent layer, and further enhancing the reproducibility of the measurement accuracy of a biosensor to be obtained, the foregoing amount is preferably 11 ng or more. From the same viewpoint, the foregoing amount is preferably 17 ng or less, 15 ng or less, 14 ng or less, or 13 ng or less. In one or more embodiments, the ejected amount of the reagent solution for the second or subsequent layers is 20 ng to 28 ng per one droplet, and from the viewpoint of further reducing irregularities in the concentrations and amounts of reagent components in the reagent layer, and further enhancing the reproducibility of the measurement accuracy of a biosensor to be obtained, the foregoing amount is preferably 21 ng or more. From the same viewpoint, the foregoing amount is preferably 26 ng or less, 25 ng or less, or 24 ng or less. In one or more embodiments, the ejected amount of the reagent solution per one droplet for the second or subsequent layer may be the same or different.

In one or more embodiments, the formation of the reagent layer includes ejecting droplets of the reagent solution for the Nth layer (N is a natural number of 1 or greater) at predetermined pitches, and ejecting droplets of the reagent solution for the (N+1)th layer on the dried droplets of the Nth layer. In one or more embodiments, N is a natural number of 1, 2, 3, 4, 5, 6, 7, 8, 9, 10, or greater.

From the viewpoint of further reducing irregularities in the concentrations and amounts of the components in the reagent layer and further enhancing the reproducibility of the measurement accuracy of a biosensor to be obtained, the formation of the reagent layer includes repeating the ejection of droplets of the reagent solution until droplets of at least the fourth layer are formed.

In one or more embodiments, the formation of the reagent layer includes a step for drying droplets, between the step of ejecting droplets for the Nth layer and the step of ejecting droplets for the (N+1)th layer. In a case where the ejection of droplets for the Nth layer and the ejection of droplets for the (N+1)th layer are carried out by different ejection devices, the time for carrying the base material (i.e. for transfer of the base material between different ejection devices) may be the time for drying the droplets.

In one or more embodiments, the pitch at which the droplets of the reagent solution are ejected (the distance between the centers of adjacent droplets) is 50 µm to 150 µm. From the viewpoint of further reducing irregularities in the concentrations and amounts of reagent components in the reagent layer, and further enhancing the reproducibility of the measurement accuracy of a biosensor to be obtained, the pitch is preferably 60 µm to 80 µm, and more preferably 65 µm to 75 µm. In one or more embodiments, the "center of a droplet" in the present disclosure refers to the center of a circle that, after a droplet of the reagent solution is formed on the conductive part and is dried, circumscribes the circumference of the dried droplet.

In one or more embodiments, the pitches at which the droplets are ejected may be fixed pitches or variable pitches. From the viewpoint of further reducing irregularities in the concentrations and amounts of reagent components in the reagent layer and further enhancing the reproducibility of the measurement accuracy of a biosensor to be obtained, the pitches are preferably fixed pitches.

In one or more embodiments, the pitches at which the droplets of the reagent solution are ejected may be different or the same between the layers. From the viewpoint of further reducing irregularities in the concentrations and amounts of reagent components in the reagent layer and further enhancing the reproducibility of the measurement accuracy of a biosensor to be obtained, the pitches are preferably the same between the layers.

In one or more embodiments, the production method of the present disclosure may also include preparing a spacer with a notch and preparing a cover in which an air hole is formed, and may additionally include placing the spacer on the substrate such that the formed reagent layer is positioned inside the notch of the spacer and placing the cover on the spacer such that the air hole of the cover is positioned directly above the notch of the spacer.

The electrodes, substrate, position of the reagent layer, and reagent in the production method of the present disclosure are the same as those in the biosensor of the present disclosure.

### (Measurement method)

The present disclosure relates, in another aspect, to a method for electrochemically measuring a measurement target in a sample using the biosensor of the present disclosure, the method comprising: bringing a sample into contact with the reagent layer of the biosensor; applying a voltage across the electrodes of the biosensor; and measuring an electric signal generated by the application of the voltage.

As used in the present disclosure, the phrase "electrochemically measuring" refers to performing a measurement using an electrochemical measurement method, which may include, in one or more embodiments, an amperometric method, a potentiometric method, a coulometric method, and the like. In one or more embodiments, an example of the amperometric method is a method in which the current value generated during the oxidation of a reduced electron mediator by the application of the voltage is measured.

The voltage to be applied is not particularly limited, but in one or more embodiments, it is 10 mV or more or 50 mV or more, and preferably 100 mV or more. In addition, in one or more embodiments, the voltage to be applied is 1000 mV or less or 700 mV or less, and preferably 600 mV or less, 500 mV or less, 400 mV or less, or 300 mV or less.

Hereinafter, embodiments of the present disclosure will be described with reference to the drawings. Note that the present invention is not limited to the embodiments given below.

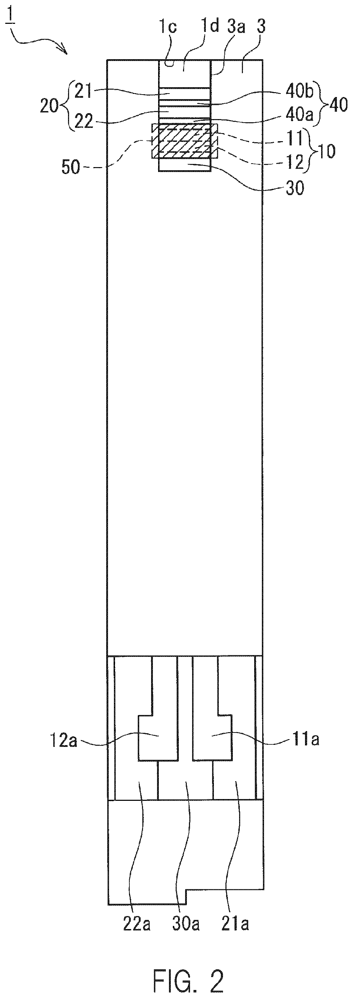

FIGS. 1 and 2 are schematic configuration diagrams for illustrating the configuration of the biosensor of the present disclosure. In FIGS. 1 and 2, the upper side is the upstream side (sample supply port side), and the lower side is the downstream side (the side to be inserted into a measurement apparatus).

As shown in FIGS. 1 and 2, a biosensor 1 includes a substrate 2, a conductive part (described later), a reagent layer 50, a spacer 3, and a cover (not shown). The conductive part is formed on one main surface of the substrate 2, and the reagent layer 50 is formed on one electrode pair 10 of the conductive part. The spacer 3 has a rectangular notch 3a and is placed on the substrate 2 such that a portion of the conductive part and a portion of the reagent layer 50 are positioned inside the notch 3a. The cover has an air hole (not shown) and is placed on the spacer 3 such that the air hole is positioned directly above the notch 3a. A space formed by the substrate 2, the notch 3a of the spacer 3, and the cover serves as a flow channel 1d, and an opening serves as a sample supply port 1c.

Polyethylene terephthalate (PET) or the like can be suitably used as the material of the substrate. In the present embodiment, the size of the substrate 2 is 7 mm in the lateral direction (width), 30 mm in the longitudinal direction (length), and 250 µm in thickness. The flow channel 1d has a width of 2 mm and a length of 4 mm. The reagent layer 50 has a width (in the lateral direction of the biosensor) of 2700 µm and a length (in the longitudinal direction of the biosensor) of 1000 µm.

The conductive part is constituted by a first electrode pair 10, a second electrode pair 20, and a blood sensing electrode 30. The first electrode pair 10 is constituted by a first working electrode 11 and a first counter electrode 12, and the second electrode pair 20 is constituted by a second working electrode 21 and a second counter electrode 22. In the flow channel 1d, the second working electrode 21, the second counter electrode 22, the first working electrode 11, the first counter electrode 12, and the blood sensing electrode 30 are arranged in this order from the upstream side, such that each electrode is exposed in a rectangular shape parallel to the lateral direction of the substrate 2. Gaps (non-conductive regions) 40b and 40a are provided between the second working electrode 21 and the second counter electrode 22, and between the second counter electrode 22 and the first working electrode 11, respectively. The first counter electrode 12 is in contact with the first working electrode 11 on the upstream side and with the blood sensing electrode 30 on the downstream side (on a face opposite to the first working electrode 11), but is insulated from these electrodes. In the present embodiment, the conductive part (electrodes) is formed by sputtering a nickel-vanadium alloy, and the electrodes can be insulated from each other by drawing a predetermined electrode pattern with a laser beam (trimming). The gaps (non-conductive regions) 40b and 40a are regions insulated by drawing a rectangular pattern with a laser beam.

The widths (lengths in the longitudinal direction of the substrate 2) of the electrodes (first working electrode 11, first counter electrode 12, second working electrode 21, and second counter electrode 22) constituting the first electrode pair 10 and the second electrode pair 20, as well as the gap 40a, are all 400 µm, and the width (length in the longitudinal direction of the substrate 2) of the gap 40b is approximately 200 µm.

Each electrode is connected to a lead portion, and the lead portions 21a of the second working electrode 21, 22a of the second counter electrode 22, 11a of the first working electrode 11, 12a of the first counter electrode 12, and 30a of the blood sensing electrode 30 each extend parallel to the longitudinal direction of the substrate 2, from one end portion to the other end portion of the substrate 2. Each electrode is covered by the spacer 3 in a region 1a that extends from the upstream end of the substrate 2 to a position near the downstream end. However, in a downstream end portion, each electrode is not covered and is exposed, and this portion serves as a connector region 1b to be inserted into an insertion port of the measurement apparatus. In the connector region 1b, the lead portion 21a of the second working electrode 21, the lead portion 22a of the second counter electrode 22, the lead portion 11a of the first working electrode 11, the lead portion 12a of the first counter electrode 12, and the lead portion 30a of the blood sensing electrode 30 each serve as an exposed contact.

The reagent layer 50 is formed by ejecting droplets of the reagent solution using an inkjet printer. The reagent layer 50 of the present embodiment has dimensions (reagent layer formation region) of 2700 µm in the lateral direction of the substrate 2 and 1000 µm in the longitudinal direction of the substrate 2.

As shown in FIG. 2 (but not shown in FIG. 1), the reagent layer 50 is formed on the conductive part exposed in the flow channel 1d. Specifically, the reagent layer 50 is formed such that the reagent covers substantially the entire surface of the first working electrode 11, a region on the surface of the first counter electrode 12 extending downstream from an edge that is in contact with the first working electrode 11 to an extent corresponding to at least 70% of the surface of the first working electrode 11, and a region extending upstream by at least 50 µm from an edge of the first working electrode 11 on the upstream side (opposite to the first counter electrode 12), or in other words, a region on the surface of the non-conductive region (gap 40a) extending upstream from an edge that is in contact with the first working electrode 11 to an extent corresponding to at least 12.5% of the surface of the first working electrode 11.

The reagent layer 50 can be formed on a portion of the conductive part that may be exposed in the flow channel 1d (notch 3a) after the spacer 3 is placed. However, the reagent layer 50 may also be formed on a portion of the conductive part that is (or may be) located outside the flow channel 1d (notch 3a), as shown in FIG. 2. This can make it possible for the reagent layer to be positioned on predetermined regions of the working electrode and the counter electrode in the flow channel, even if a production error (e.g., deviation in the location where the notch is formed, positioning misalignment of the spacer, or the like) occurs during the biosensor production process.

The reagent layer 50 may or may not be formed on the second working electrode 21 and the second counter electrode 22, which constitute the second electrode pair. A reagent layer with a composition different from that of the reagent layer 50 may be formed on the second working electrode 21 and the second counter electrode 22, which constitute the second electrode pair.

The formation of the reagent layer 50 using an inkjet printer can be performed in the following manner: with the interval between ejected droplets (center-to-center distance between dots (ejected droplets of the reagent solution)) being set to 70 µm, droplets of the reagent solution are evenly ejected to a total of 546 locations, specifically, 39 locations in the lateral direction of the substrate, 2 × 14 locations in the longitudinal direction of the substrate 2, within the reagent layer formation region, to form a dot group including a plurality of reagent dots (ejected droplets of the reagent solution) (e.g., 546 reagent dots (39 dots × 14 dots)), and the formation of such a dot group is then repeatedly performed. The formation of a dot group is preferably repeated three or more times (i.e. to form three or more layers), and more preferably five or more times (i.e. to form five or more layers), in order to facilitate the formation of a reagent layer with an average thickness of 4 µm or more, enhance the uniformity of the thickness of the reagent layer to be formed, and further improve measurement accuracy. From the viewpoint of enhancing the uniformity of the thickness of the reagent layer to be formed and further improving measurement accuracy, it is preferable that the locations to which droplets of the reagent solution are ejected in dot groups formed in odd-numbered instances (in odd-numbered layers) and those in dot groups formed in even-numbered instances (in even-numbered layers) are different.

Note that during the formation of the reagent layer 50, the interval between ejected droplets of the reagent solution and the number (locations) of ejected droplets of the reagent solution in a dot group (a number of ejected droplets/dot group) are not limited to the above-described form and can be appropriately determined based on the size (area) of the reagent layer to be formed, the droplet volume to be ejected, the composition of the reagent solution to be ejected, and the like.

From the viewpoint of enhancing the uniformity of the thickness of the reagent layer to be formed and further improving measurement accuracy, a certain time period for allowing the ejected droplets of the reagent solution to dry may be provided between the formation of one dot group and the formation of the next dot group. Regarding the time period for drying, in one or more embodiments, the time required for transport of the substrate for allowing droplets of the reagent solution to be ejected to predetermined locations from a nozzle of an inkjet head may be regarded as the drying time, or alternatively, a separate time period may be provided for a drying process, independent of the transport process. When a plurality of inkjet printers are used to form the reagent layer, the time required for transport between the inkjet printers may be utilized for the drying process.

Hereinafter, a method for forming a reagent layer using an inkjet printer will be described, using the example of repeating the formation of a dot group five times (i.e. the formation of five layers).

First, a reagent solution is prepared. The composition of the reagent solution can be appropriately determined based on the type of reagent layer to be formed and the type of biosensor to be produced.

In one or more embodiments, the amount of oxidoreductase added in the reagent solution is 1 KU/mL to 200 KU/mL. In one or more embodiments, the amount of oxidoreductase added in the reagent liquid is 5 KU/mL or more, 10 KU/mL or more, 15 KU/mL or more, 20 KU/mL or more, 25 KU/mL or more, or 30 KU/mL or more. In one or more embodiments, the amount of oxidoreductase added in the reagent liquid is 100 KU/mL or less, 90 KU/mL or less, 80 KU/mL or less, 70 KU/mL or less, 60 KU/mL or less, or 55 KU/mL or less.

In one or more embodiments, the amount of electron mediator added in the reagent solution is 1 mM to 1000 mM. In one or more embodiments, the amount of electron mediator added in the reagent solution is 2 mM or more, 5 mM or more, 10 mM or more, 50 mM or more, 100 mM or more, 200 mM or more, or 500 mM or more. In one or more embodiments, the amount of electron mediator added in the reagent solution is 900 mM or less, 800 mM or less, or 700 mM or less. In one or more embodiments, the amount of buffer added in the reagent solution is 10 mM to 1000 mM. In one or more embodiments, the amount of buffer added in the reagent solution is 20 mM or more, 50 mM or more, 100 mM or more, 150 mM or more, or 200 mM or more. In one or more embodiments, the amount of buffer added in the reagent liquid is 900 mM or less, 800 mM or less, 700 mM or less, 600 mM or less, or 500 mM or less.

In one or more embodiments, the amount of amino acid added in the reagent solution is 0.1 mass% to 20 mass% or 0.5 mass% to 10 mass%. In one or more embodiments, the amount of surfactant added in the reagent solution is 0.1 mass% to 20 mass% or 0.5 mass% to 10 mass%.

A dot group of a first layer (a first dot group) is formed using the prepared reagent solution.

The reagent solution for the first dot group is ejected at an ejection volume that prevents the droplets of the reagent solution from coalescing (combining) with each other after being ejected from the nozzle. For example, the ejection volume of the reagent solution can be set between 10 ng and 18 ng (e.g., 12 ng). With this volume, it is possible to suppress the combining of the droplets of the reagent solution ejected onto the conductive part (substrate) into a single droplet. Thus, each droplet is placed on the conductive part (substrate) as an independent single droplet. The size of each droplet (after drying, major axis) is 50 µm to 70 µm.

After the droplets constituting the first dot group are ejected, it is preferable to allow the reagent of the first dot group to dry before ejecting the reagent solution for a dot group of a second layer (second dot group). As a result of allowing the reagent of the first dot group to dry before ejecting the next droplets of the reagent solution onto the first dot group, the newly ejected droplets of the reagent solution dissolve a portion of the reagent of the first dot group, and the newly ejected droplets dry together with the dissolved reagent. This enables further reduction in the unevenness of the reagent and further improvement in measurement accuracy. Drying can be performed at room temperature (with a humidity of 50% or less) for 20 seconds or longer.

Next, the dot group of the second layer (second dot group) is formed on top of the dried first dot group.

The reagent solution for the second dot group is ejected to locations different from those to which the reagent solution for the first dot group has been ejected, since this can further reduce the unevenness of the reagent and further improve measurement accuracy. For example, it is preferable to eject the droplets of the reagent solution to the intersections connecting the centers of adjacent reagent dots of the first dot group.

It is preferable that the reagent solution for the second dot group is ejected in such a manner that each droplet of the reagent solution ejected from the nozzle overlaps at least a portion of an adjacent droplet of the reagent solution, since this can further reduce the unevenness of the reagent and further improve measurement accuracy. For example, the ejection volume of the droplets can be set between 20 ng and 28 ng (e.g., 23 ng). With this volume, each droplet of the reagent solution ejected from the nozzle can be allowed to dry while overlapping an adjacent droplet of the reagent solution.

After the droplets constituting the second dot group are ejected, it is preferable to allow the reagent of the second dot group to dry before ejecting the reagent solution for a dot group of a third layer. As a result of allowing the reagent of the second dot group to dry before the next droplets of the reagent solution are ejected onto the second dot group, the newly ejected droplets of the reagent solution dissolve a portion of the reagent of the first and second dot groups, and the newly ejected droplets dry together with the dissolved reagent. This enables further reduction in the unevenness of the reagent and further improvement in measurement accuracy. Drying can be performed in a similar manner to that of the first dot group.

Next, the dot group of the third layer (third dot group) is formed on top of the dried second dot group.

The reagent solution for the third dot group is ejected to locations substantially the same as those to which the reagent solution for the first dot group has been ejected, since this can further reduce the unevenness of the reagent and further improve measurement accuracy. The ejection volume of the droplets and the drying method can be similar to those of the second dot group.

Next, a dot group of a fourth layer (fourth dot group) is formed on top of the dried third dot group.

The reagent solution for the fourth dot group is ejected to locations substantially the same as those to which the reagent solution for the second dot group has been ejected, since this can further reduce the unevenness of the reagent and further improve measurement accuracy. The ejection volume of the droplets and the drying method can be similar to those of the second dot group.

Finally, a dot group of a fifth layer (fifth dot group) is formed on top of the dried fourth dot group.

The reagent solution for the fifth dot group is ejected to locations substantially the same as those to which the reagent solution for the first or third dot group has been ejected, since this can further reduce the unevenness of the reagent and further improve measurement accuracy. The ejection volume of the droplets and the drying method can be similar to those of the third dot group.

The formation of those dot groups can form a reagent layer with an average thickness with 4 µm or more and 12 µm or less, 5 µm or more and 12 µm or less, 6 µm or more and 12 µm or less, 7 µm or more and 12 µm or less, 8 µm or more and 12 µm or less, 9 µm or more and 12 µm or less, or 9.5 µm or more and 12 µm or less.

Subsequently, a spacer 3 with a notch 3a is placed on the substrate 2 on which the reagent layer 50 has been formed. The spacer 3 is placed on the substrate 2 such that a portion of the conductive part (the first working electrode 11, the first counter electrode 12, the second working electrode 21, the second counter electrode 22, and the blood sensing electrode 30) on which the reagent layer 50 is placed is positioned (exposed) inside the notch 3a.

Finally, a cover (not shown) with an air hole is placed on the spacer 3.

When PMMA is used as the material of the spacer 3, the adhesiveness of PMMA allows the substrate 2 and the cover to adhere to each other. It is also possible to stack the spacer 3 and the cover in advance, place this stack on the substrate 2, and allow them to adhere to each other.

An example of the method for electrochemically measuring a measurement target in a sample using the biosensor 1 will be described using a case where the sample is whole blood and the measurement target is glucose as an example.

First, the whole blood sample is brought into contact with the sample supply port 1c of the biosensor 1. When the whole blood sample is drawn into the sample supply port 1c of the biosensor 1, the sample flows downstream through the flow channel 1d due to capillary force, passing sequentially through the second working electrode 21, the second counter electrode 22, the first working electrode 11, the first counter electrode 12 and the blood sensing electrode 30. At this time, when the whole blood sample reaches the reagent layer 50, the reagent components (oxidoreductase, electron mediator, etc.) contained in the reagent layer 50 are dissolved by the whole blood sample.

Then, when a positive potential is applied across the electrodes, electrons are exchanged between the electron mediator present in the reagent layer 50 and the first working electrode 11 located beneath the reagent layer 50, resulting in an oxidation current. Based on this, the glucose concentration can be measured. The voltage applied is, in one or more embodiments, 10 mV to 1000 mV, and preferably 100 mV to 600 mV.

The embodiment above has been described using the form of the biosensor and the measurement method in the case where the sample is blood (e.g., whole blood) as examples. However, the present disclosure is not limited to this. Various biological samples other than blood, such as urine, can also be measured in the same manner.

The present disclosure may relate to one or more non-limiting embodiments described below.
(1) A biosensor including:
   a substrate with a conductive part formed on one main surface, the conductive part including an electrode pair having a working electrode and a counter electrode;
   a reagent layer placed on the working electrode and the counter electrode; and
   a flow channel for allowing a sample to flow from a sample supply port to the reagent layer,
   wherein the reagent layer is formed to cover the entirety of the region of the working electrode within the flow channel and extend at least 50 µm from upstream and downstream edges of the working electrode, and to cover a region, of the region of the counter electrode within the flow channel, that corresponds to at least 70% of the region of the working electrode, and
   the reagent layer has an average thickness of 4 µm to 12 µm.
(2) A biosensor including:
   a substrate with a conductive part formed on one main surface, the conductive part including an electrode pair having a working electrode and a counter electrode;
   a reagent layer placed on the working electrode and the counter electrode; and
   a flow channel for allowing a sample to flow from a sample supply port to the reagent layer,
   wherein the reagent layer is formed to cover the entirety of the surface of the working electrode within the flow channel and at least a portion of the surface of the counter electrode within the flow channel, and to extend upstream from an upstream edge of the working electrode and downstream from a downstream edge of the working electrode, with extents of the upstream and downstream extensions each corresponding to at least 12.5% of the surface area of the working electrode within the flow channel, and a region of the counter electrode covered by the reagent layer corresponds to at least 70% of the surface area of the working electrode within the flow channel, and
   the reagent layer has an average thickness of 4 µm to 12 µm.
(3) The biosensor according to clause (1) or (2),
   wherein the reagent layer extending from the downstream edge of the working electrode has a larger area than the reagent layer extending from the upstream edge of the working electrode.
(4) The biosensor according to any one of clauses (1) to (3),
   wherein the working electrode and the counter electrode extend in a direction perpendicular to a direction of sample supply in the flow channel, and
   the working electrode is formed upstream of and adjacent to the counter electrode.
(5) The biosensor according to any one of clauses (1) to (4),
   wherein the reagent layer extends within a range of 100 µm or more and less than 150 µm from the edges of the working electrode.
(6) The biosensor according to any one of clauses (1) to (5),
   wherein the reagent layer is formed through dot printing.
(7) A method for producing a biosensor including a conductive part and a reagent layer within a flow channel, the method including:
   preparing a substrate with the conductive part formed on one main surface, the conductive part including an electrode pair having a working electrode and a counter electrode; and
   forming the reagent layer on the working electrode and the counter electrode,
   wherein the forming of the reagent layer includes dot printing a reagent solution such that the entirety of a region of the working electrode within the flow channel, regions extending at least 50 µm from upstream and downstream edges of the working electrode, and a region, of the region of the counter electrode within the flow channel, that corresponds to at least 70% of the region of the working electrode within the flow channel are covered by a reagent, and that the reagent layer has an average thickness of 4 µm to 12 µm.
(8) The method according to clause (7), for producing the biosensor according to any one of clauses (1) to (6).
(9) The method according to clause (7) or (8),
   wherein forming the reagent layer comprises repeatedly dot printing the reagent solution on the conductive part to form the reagent layer in which the reagent is stacked three-dimensionally.
(10) The method according to any one of clauses (7) to (9),
   wherein forming the reagent layer comprises:
   dot printing the reagent solution for an Nth layer at predetermined pitches;
   drying the droplets of the reagent solution, and
   dot printing the reagent solution for an (N+1)th layer on the dried droplets of the Nth layer; wherein "N" is a natural number of 1 or greater; and
   wherein dot printing the reagent solution for the (N+1)th layer is performed so that the droplets at least partially cover the dried droplets in the Nth layer.
(11) The method according to any one of clauses (7) to (10),
   wherein forming the reagent layer comprises repeatedly dot printing the reagent solution until at least the fourth layer is stacked.

Hereinafter, the present disclosure will be described in greater detail by means of examples; however, these examples are illustrative, and the present disclosure is not limited to these examples.

### EXAMPLE

### (Biosensor Production 1)

(1) As shown in FIG. 1, a substrate 2 (PET, 250 µm thick, 30 mm long × 7 mm wide) with a conductive part formed on one main surface was prepared.

The conductive part was constituted by a first electrode pair 10, a second electrode pair 20, and a blood sensing electrode 30. The first electrode pair 10 was constituted by a first working electrode 11 and a first counter electrode 12, and the second electrode pair 20 was constituted by a second working electrode 21 and a second counter electrode 22. The second working electrode 21, the second counter electrode 22, the first working electrode 11, and the first counter electrode 12 were arranged in this order on one end portion of the substrate 2 in the longitudinal direction, parallel to the lateral direction of the substrate 2. Gaps (non-conductive regions) 40b and 40a were provided between the second working electrode 21 and the second counter electrode 22, and between the second counter electrode 22 and the first working electrode 11, respectively. The first working electrode 11 was in contact with the first counter electrode 12, and the opposite face of the first counter electrode 12, relative to the first working electrode 11, was also in contact with the blood sensing electrode 30. Each electrode was connected to a lead portion, and the lead portions 21a of the second working electrode 21, 22a of the second counter electrode 22, 11a of the first working electrode 11, 12a of the first counter electrode 12, and 30a of the blood sensing electrode 30 each extended parallel to the longitudinal direction of the substrate 2, from one end portion to the other end portion of the substrate 2.

The widths (lengths in the longitudinal direction of the substrate 2) of the electrodes (first working electrode 11, first counter electrode 12, second working electrode 21, and second counter electrode 22) constituting the first electrode pair 10 and the second electrode pair 20, as well as the gap 40a, were all 400 µm, and the width (length in the longitudinal direction of the substrate 2) of the gap 40b was approximately 200 µm.

(2) Next, a reagent solution with the following composition was prepared.

### (Composition of Reagent Solution)

FAD-dependent Glucose Dehydrogenase (trade name: Glucose Dehydrogenase "Amano 8", MW: 180,000, manufactured by Amano Enzyme Inc.): 41 KU/mL
Ru complex (Ru(NHs)sCls, manufactured by LT Metal Co. Ltd.): 626 mM
1-Methoxy PES (1-methoxy-5-ethylphenazinium ethylsulfate, manufactured by Dojindo Laboratories): 824 µg/mL
0.4 M Phosphate buffer (pH 7.0): 0.29 mM
Glycine: 2 mass%
CHAPS: 2 mass%
Antifoaming agent: 0.04 mass%

(3) Next, a reagent layer was formed on the substrate 2 on which the above-described conductive part was formed.

The reagent layer was formed by dot printing the reagent solution using an inkjet apparatus under conditions at room temperature and at a humidity of 50% or less, according to the following procedure. A reagent layer formation region (the area where the reagent was dot-printed) was rectangular and had a length of 2700 µm in the lateral direction of the substrate 2 and a length of 1000 µm in the longitudinal direction of the substrate 2. The reagent layer was formed in nine different patterns (type 1 to type 9), where the reagent layer 50 covered the entire surface of the first working electrode 11 and at least a portion of the first counter electrode 12, while the position where the reagent layer was formed was shifted in 50 µm increments in the longitudinal direction of the substrate 2, as shown in Table 1 below.

### <Procedure>

1. The reagent solution was filled into a storage section of the inkjet apparatus. Droplets of the reagent solution were ejected onto the substrate on which the conductive part was formed, under the following conditions, and allowed to dry for 30 seconds. Thus, a first layer of the reagent (first dot group) was placed. The diameter (after drying) of each dot of the dot-printed reagent was 50 µm to 70 µm.

### (Ejection Conditions (First Dot Group))

Ejection volume per droplet: 12 ng (10 pL)
Interval between Droplets (center-to-center distance between ejected droplets): 70 µm
Number of ejections: 546 locations (39 locations in the lateral direction of the substrate, 2 × 14 locations in the longitudinal direction of the substrate 2)

2. Next, a second layer of the reagent (second dot group) was placed on top of the first dot group by ejecting droplets of the reagent solution under the following conditions and allowing them to dry for 30 seconds. For the second layer, droplets were ejected such that the location to which each droplet of the reagent solution was ejected (the center of each droplet) was approximately at the center of a square formed by the centers of four adjacent reagent dots in the first dot group, with these centers at the respective apexes of the square. The diameter (after drying) of each dot of the dot-printed reagent was 90 µm to 100 µm.

### (Ejection Conditions (Second Dot Group))

Ejection volume per droplet: 23 ng (18 pL)
Interval between Droplets (center-to-center distance between ejected droplets): 70 µm
Number of ejections: 546 locations (39 locations in the lateral direction of the substrate, 2 × 14 locations in the longitudinal direction of the substrate 2)

3. Next, a third layer of the reagent (third dot group) was placed on top of the second dot group by ejecting droplets of the reagent solution under the following conditions and allowing them to dry. The droplets of the third dot group were ejected in the same manner as those of the second dot group, except that the locations to which the droplets of the reagent solution were ejected were almost the same as those of the first dot group.

4. Next, a fourth layer of the reagent (fourth dot group) was placed on top of the third dot group by ejecting droplets of the reagent solution and allowing them to dry, in the same manner as the second dot group.

5. Finally, a fifth layer of the reagent (fifth dot group) was placed on top of the fourth dot group by ejecting droplets of the reagent solution and allowing them to dry, in the same manner as the third dot group. Consequently, a three-dimensionally layered reagent layer (substantially a rectangular parallelepiped (or quadrangular prism)) was obtained. The thickness of the reagent layer was observed under a laser microscope (product name: VK-X3000, manufactured by Keyence Corp.). The thickness distribution of the reagent layer ranged from 8 µm to 12 µm, with an average thickness of approximately 10 µm. The amount of FAD-dependent Glucose Dehydrogenase in the obtained reagent layer was 232 KU/cm³, and the amount of electron mediator was 3.56 mmol/cm³.

(4) A spacer 3 with a notch 3a (length in the longitudinal direction of the substrate 2: 4 mm, length in the lateral direction of the substrate 2: 2 mm) was placed on the substrate 2, on which the reagent layer was formed, as shown in FIG. 2. Furthermore, a cover (not shown) with an air hole was placed on the spacer 3. A space formed by the substrate 2, the notch 3a of the spacer 3, and the cover served as a flow channel 1d.

### (Measurement of Size of Dot-Printed Reagent Dot)

The size of each reagent dot formed by a single droplet ejected from the inkjet printer was measured using a digital microscope (VHX-6000, manufactured by Keyence Corp.).

### (Glucose Current Value Measurement 1)

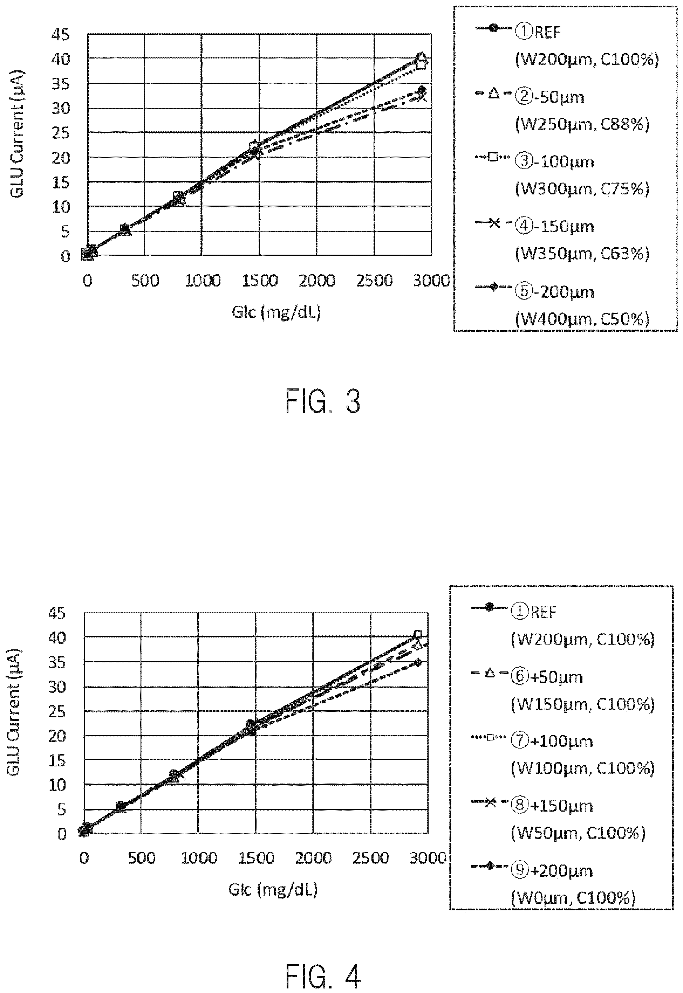

Specimens with the following various glucose (GLU) concentrations were prepared. Electrochemical measurements were performed by introducing these specimens into the prepared biosensors. In the electrochemical measurements, using a potentiostat (manufactured by ARKRAY, Inc.), a voltage of 0.2 V was applied, and the current value was measured after 4.0 seconds had elapsed. FIGS. 3 to 5 show the results. R² shown in Table 1 is the coefficient of determination for an approximate curve (linear approximation) created for each of the graphs of current values corresponding to the various biosensors shown in FIGS. 3 and 4.

### <Evaluation Conditions>

Specimens used: Whole blood specimens
GLU concentrations (mg/dl): 0, 45, 330, 800, 1500, and 3000 (all at Hct 42%)
Number of evaluations: n = 10 (for each GLU concentration)

| (Table1) | Gap 40a | | First working electrode 11 (W) | First counter electrode 12 (C) | | R² |
|---|---|---|---|---|---|---|
| | Region (length) from upstream edge of working electrode 11 (in longitudinal direction of substrate 2) | Region of reagent layer (relative to surface area of working electrode 11) | Region of reagent layer that covers surface | Region (length) from downstream edge of working electrode 11 (in longitudinal direction of substrate 2) | Region of reagent layer that covers surface of counter electrode 12 (relative to surface area of working electrode 11) | |
| type 1 | 200µm | 50% | 100% | 400µm | 100% | 0.9983 |
| type 2 | 250µm | 63% | 100% | 350µm | 88% | 0.9976 |
| type 3 | 300µm | 75% | 100% | 300µm | 75% | 0.9958 |
| type 4 | 350µm | 88% | 100% | 250µm | 63% | 0.9856 |
| type 5 | 400µm | 100% | 100% | 200µm | 50% | 0.9839 |
| type 6 | 150µm | 38% | 100% | 400µm | 100% | 0.9984 |
| type 7 | 100µm | 25% | 100% | 400µm | 100% | 0.9996 |
| type 8 | 50µm | 13% | 100% | 400µm | 100% | 0.9989 |
| type 9 | 0µm | 0% | 100% | 400µm | 100% | 0.9900 |

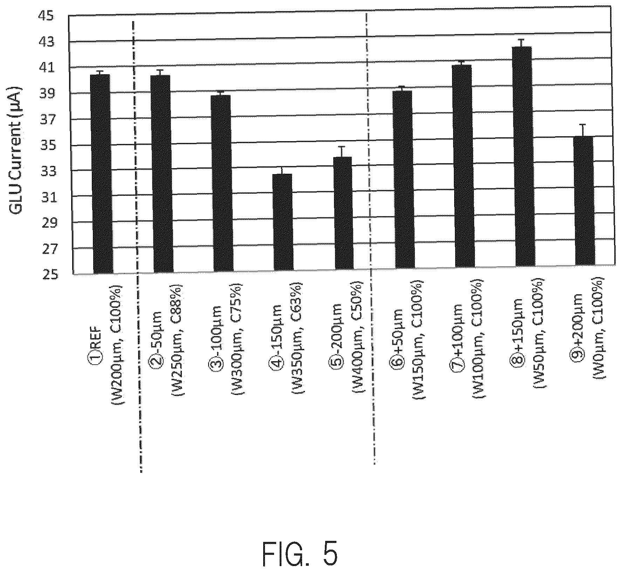

FIGS. 3 and 4 are graphs showing the relationship between glucose concentration and reaction current values, and FIG. 5 is a graph showing the reaction current values of the various biosensors measured when a specimen with a GLU concentration of 3000 mg/dl and an Hct of 42% was used. FIG. 3 shows linearity of glucose measurement in biosensors (types 1 to 5) with a different region of the reagent layer that covers the surface of the counter electrode 12. FIG. 4 shows linearity of glucose measurement in biosensors (types 1 and 6 to 9) with a different region of the reagent layer that extends from the upstream edge of working electrode 11. As shown in FIGS. 3 to 5, with biosensors where the reagent layers were formed at the positions of types 4, 5 and 9, respectively, the reaction current value decreased at high glucose concentrations, resulting in insufficient linearity of glucose measurement. In contrast, with biosensors where the reagent layers were formed at the positions of types 1 to 3 and 6 to 8, no decrease in the reaction current value was observed, and high linearity of glucose measurement was achieved.

### (Biosensor Production 2)

Seven types of biosensors (types 1 to 3, 5 to 7, and 9) with different reagent layer positions were produced by preparing a substrate with a conductive part formed thereon in the same manner as described in Biosensor Production 1, and by placing the first to third layers of the reagent in the same manner as described in steps 1 to 4 of <Procedure>. The obtained reagent layers had an average thickness of approximately 6 µm (thickness distribution: 4 µm to 8 µm).

### (Glucose Current Value Measurement 2)

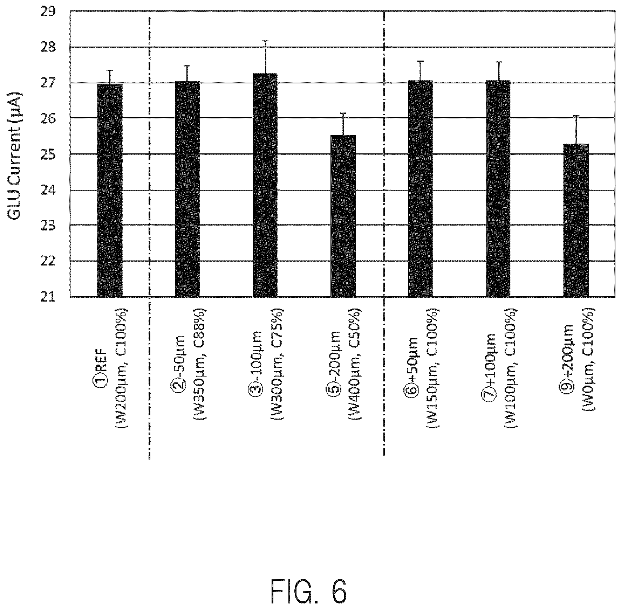

Measurements were performed in the same manner as described in Glucose Current Value Measurement 1, except that the current value after 4.0 seconds had elapsed was measured using the biosensors produced in Biosensor Production 2. Of the results obtained, the results using a specimen with a GLU concentration of 1500 mg/dl × Hct 42% are shown in FIG. 6.

As shown in FIG. 6, the biosensors where the reagent layers were formed at the positions of types 5 and 9 exhibited low reaction current values, whereas the other biosensors (types 1 to 3, 6, and 7) exhibited no decrease in reaction current values, suggesting that high linearity of glucose measurement was achieved.

### (Biosensor Production 3)

Seven types of biosensors (types 1 to 3, 5 to 7, and 9) with different reagent layer positions were produced by preparing a substrate with a conductive part formed thereon in the same manner as described in Biosensor Production 1, and by placing the first layer of the reagent under the ejection conditions for the second dot group in step 1 of <Procedure>. The obtained reagent layers had an average thickness of approximately 2 µm (thickness distribution: 1 µm to 5 µm).

### (Glucose Current Value Measurement 3)

Measurements were performed in the same manner as described in Glucose Current Value Measurement 1, except that the current value after 4.0 seconds had elapsed was measured using the biosensors produced in Biosensor Production 3 and using specimens with the glucose (GLU) concentrations below.

### <Evaluation Conditions>

Specimens used: Whole blood specimens
GLU concentrations (mg/dl): 0, 45, 330, 800, and 1500 (all at Hct 42%)
Number of evaluations: n = 10 (for each GLU concentration)

As a result, with the reagent layers having an average thickness of approximately 2 µm (less than 4 µm), the reagent in the reagent layer was diffused by the sample, and a sufficient amount of the reagent needed for measurement was no longer present on the working electrode. This resulted in the problem that sufficient measurement current values were not obtained for specimens with high glucose concentrations (e.g., 800 mg/dl) and, in some cases, the problem of an increase in the current value. Consequently, the measurements were unstable, resulting in poor measurement performance.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A biosensor comprising:
a substrate with a conductive part provided on one main surface, the conductive part comprising an electrode pair having a working electrode and a counter electrode;
a reagent layer provided on the working electrode and the counter electrode; and
a flow channel for allowing a sample to flow from a sample supply port to the reagent layer,
wherein the reagent layer has an average thickness of 4 µm to 12 µm, and
wherein the reagent layer is provided to cover the entirety of the region of the working electrode within the flow channel and extend at least 50 µm from upstream and downstream edges of the working electrode, and to cover a region, of the region of the counter electrode within the flow channel, that corresponds to at least 70% of the region of the working electrode within the flow channel,
and/or
wherein the reagent layer is provided to cover the entirety of the surface of the working electrode within the flow channel and at least a portion of the surface of the counter electrode within the flow channel, and to extend upstream from an upstream edge of the working electrode and downstream from a downstream edge of the working electrode, with the extents of the upstream and downstream extensions each corresponding to at least 12.5% of the surface area of the working electrode within the flow channel, and a region of the counter electrode covered by the reagent layer corresponds to at least 70% of the surface area of the working electrode within the flow channel.

2. The biosensor according to claim 1,
wherein the reagent layer is provided to cover the entirety of the region of the working electrode within the flow channel and extend at least 50 µm from upstream and downstream edges of the working electrode, and to cover a region, of the region of the counter electrode within the flow channel, that corresponds to at least 70% of the region of the working electrode within the flow channel.

3. The biosensor according to claim 1,
wherein the reagent layer is provided to cover the entirety of the surface of the working electrode within the flow channel and at least a portion of the surface of the counter electrode within the flow channel, and to extend upstream from an upstream edge of the working electrode and downstream from a downstream edge of the working electrode, with extents of the upstream and downstream extensions each corresponding to at least 12.5% of the surface area of the working electrode within the flow channel, and a region of the counter electrode covered by the reagent layer corresponds to at least 70% of the surface area of the working electrode within the flow channel.

4. The biosensor according to any one of claims 1 to 3,
wherein the reagent layer has a larger surface area extending from the downstream edge of the working electrode than the surface area of the reagent layer extending from the upstream edge of the working electrode.

5. The biosensor according to any one of claims 1 to 4,
wherein the working electrode and the counter electrode extend in a direction perpendicular to the direction of sample supply in the flow channel, and
the working electrode is provided upstream of and adjacent to the counter electrode.

6. The biosensor according to any one of claims 1 to 5,
wherein the reagent layer extends within a range of 100 µm or more and less than 150 µm from the edges of the working electrode.

7. The biosensor according to any one of claims 1 to 6,
wherein the reagent layer is provided to cover a region, of the region of the counter electrode within the flow channel, that corresponds to at least 75% of the region of the working electrode in the flow channel.

8. The biosensor according to any one of claims 1 to 7,
wherein the reagent layer is formed through dot printing.

9. The biosensor according to claim 8,
wherein the reagent layer is formed by repeatedly dot printing a reagent solution on the conductive part to form the reagent layer in which the reagent layer is stacked three-dimensionally.

10. A method for producing the biosensor according to any one of claims 1 to 9, the method comprising:
preparing a substrate with a conductive part provided on one main surface, the conductive part comprising an electrode pair having a working electrode and a counter electrode; and
forming a reagent layer on the working electrode and the counter electrode.

11. The method according to claim 10,
wherein forming the reagent layer comprises dot printing a reagent solution.

12. The method according to claim 11,
wherein forming the reagent layer comprises repeatedly dot printing the reagent solution on the conductive part to form the reagent layer in which the reagent layer is stacked three-dimensionally.

13. The method according to any one of claims 10 to 12,
wherein forming the reagent layer comprises:
dot printing the reagent solution for an Nth layer at predetermined pitches;
drying the droplets of the reagent solution, and
dot printing the reagent solution for an (N+1)th layer on the dried droplets of the Nth layer; wherein "N" is a natural number of 1 or greater; and
wherein dot printing the reagent solution for the (N+1)th layer is performed so that the droplets at least partially cover the dried droplets in the Nth layer.

14. The method according to any one of claims 10 to 13,
wherein forming the reagent layer comprises repeatedly dot printing the reagent solution until at least a fourth layer is stacked.

15. A method for electrochemically measuring a measurement target in a sample using the biosensor according to any one of claims 1 to 9, the method comprising:
bringing the sample into contact with the reagent layer of the biosensor;
applying a voltage across the electrodes of the biosensor; and
measuring an electric signal generated by the application of the voltage.
